# EUROPEAN PATENT SPECIFICATION

(11) **EP 3 990 055 B1**
(45) Date of publication and mention of the grant of the patent: **02.04.2025**
(21) Application number: 20730079.9
(22) Date of filing: 08.06.2020
(51) Int. Cl.: A61M 5/20, A61M 5/315, G09B 23/28

(54) **DRIVE MECHANISM FOR A MEDICAMENT DELIVERY TRAINING DEVICE**
ANTRIEBSMECHANISMUS FÜR EINE ARZNEIMITTELABGABETRAININGSVORRICHTUNG
MÉCANISME D'ENTRAÎNEMENT POUR DISPOSITIF DE FORMATION À L'ADMINISTRATION DE MÉDICAMENTS

(30) Priority: 26.06.2019 EP 19182613
(43) Date of publication of application: 04.05.2022
(73) Proprietor: SHL Medical AG, 6302 Zug (CH)
(72) Inventor: YIN, Ming-Ting, Taoyuan City, Taiwan 338 (TW)
(86) International application number: PCT/EP2020/065851
(87) International publication number: WO 2020/259997

(56) References cited:
- WO-A1-2017/140452
- WO-A1-2017/144211
- WO-A1-2017/153077

## Description

### TECHNICAL FIELD

The present disclosure generally relates to medicament delivery devices. In particular, it relates to drive mechanism for a medicament delivery training device and to a medicament delivery training device comprising such a drive mechanism.

### BACKGROUND

Medicament delivery devices such as auto-injectors nowadays provide possibilities for the users themselves to handle medicament delivery in an easy, safe and reliable manner.

Before a user commences a drug administration programme by means of an auto-injector, it may be valuable for the user to undergo training to learn how to administer a drug properly by means of a specific auto-injector. A training device may be used for this purpose. It is known to provide a feedback mechanism for indicating to the user when the appropriate volume of medicament has been injected by the auto-injector. There are feedback mechanism that indicate the user when the injector can be removed from an injection site.

WO2018/082887 discloses a feedback mechanism for an injection device configured to deliver a medicament to a user. The feedback mechanism comprises a piston and a fluid chamber. The piston is adapted to move into the fluid chamber during use of the injection device. The feedback mechanism also has a damper arranged to damp movement of the piston. An indicator is arranged to provide feedback to the user after the piston has moved a pre-determined distance into the fluid chamber.

To conduct an exact imitation of the auto-injector functions, it is desired that the trainer is also able to produce signals for indicating the different steps in the injecting process for the user as the real auto-injectors. This will improve a training efficiency. Such feedback mechanisms can be different, produce different audio, tactical and visual signals and indicate a beginning or an end of the imitated injection process.

Furthermore, it is advantageous if the trainer device could be used a number of times for a training purpose. Therefore, it requires a possibility to be reset after a training operation for a multiple usage. Exemplary automatic injection training devices are known from WO 2017/153077 A1 and WO 2017/144211 A1.

### SUMMARY

An object of the present disclosure is to provide a drive mechanism for a medicament delivery training device which solves or at least mitigates problems of the prior art.

There is hence according to a first aspect of the present disclosure provided a drive mechanism for a medicament delivery training device, the delivery mechanism comprising: a plunger rod having a proximal end and a distal end, a stopper provided on the proximal end of the plunger rod, and a container having an inner chamber, a proximal container end, and an open distal container end leading into the inner chamber, wherein the inner chamber has a distal portion and a proximal portion, wherein in an initial state of the delivery mechanism the plunger rod is axially fixedly arranged in a first position in which the stopper is arranged distally relative to the proximal portion, the plunger rod being biased towards the proximal container end, and wherein the plunger rod is configured to be released from the first position, causing the plunger rod to move towards the proximal container end and the stopper to impact with a stopper contact surface, resulting in an initial signal, and to subsequently move into the proximal portion.

A medicament delivery training device, for which the drive mechanism is designed for, may thereby simulate medicament administration of a real medicament delivery device. In particular, a user is due to the initial signal able to learn and understand when the medicament administration process is commenced during medicament administration.

The initial signal may be an initial sound such as an initial click.

According to one embodiment the stopper has an outer stopper diameter, the distal portion has a distal portion inner diameter and the proximal portion has a proximal portion inner diameter which is smaller than the distal portion inner diameter and the outer stopper diameter, the distal portion transitioning to the proximal portion via the stopper contact surface. The stopper hence impacts with the stopper contact surface because the outer stopper diameter is larger than the proximal portion inner diameter.

One embodiment comprises a holding structure provided with a radially flexible arm, wherein the plunger rod is provided with a radial recess configured to engage with the radially flexible arm to maintain the plunger rod axially fixed in the distal portion.

One embodiment comprises an actuator sleeve configured to receive the holding structure, the actuator sleeve being configured to move between a proximal position and a distal position, wherein in the proximal position the actuator sleeve is configured to urge the radially flexible arm radially inwards to engage with the radial recess.

According to one embodiment when the actuator sleeve is in the distal position the actuator sleeve is configured to enable the radially flexible arm to move radially outwards to disengage from the radial recess, thereby releasing the plunger rod from the first position.

According to one embodiment the actuator sleeve is biased towards the proximal position.

One embodiment comprises a delivery member cover configured to move linearly relative to the container, between an initial position and an activating position, wherein the delivery member cover is configured to move the actuator sleeve from the proximal position to the distal position when moved from the initial position to the activating position.

One embodiment comprises a housing configured to receive the container, wherein the housing has an inner stop structure configured to stop proximal movement of the plunger rod in the inner chamber, causing the stopper to impact with the stop structure, resulting in a subsequent signal.

The subsequent signal may be a subsequent audible sound such as a click.

A user may thereby be able to learn and understand when a medicament administration process has been finalised.

According to one embodiment the stop structure comprises a plurality of radially inwards extending protrusions.

According to one embodiment the container is provided with a proximal engagement structure configured to engage with the housing.

According to one embodiment the radially inwards extending protrusions and the proximal engagement structure are arranged to form a central proximal opening into the container.

According to one embodiment the stopper is made of a resilient material.

According to one embodiment the proximal portion inner diameter is dimensioned to cause friction between the stopper and the inner surface of the proximal portion to control a driving speed of the plunger rod.

There is according to a second aspect of the present disclosure provided a medicament delivery training device comprising a delivery mechanism according to the first aspect.

According to one embodiment the drive mechanism is configured to be actuated mechanically or electrically.

Generally, all terms used in the claims are to be interpreted according to their ordinary meaning in the technical field, unless explicitly defined otherwise herein. All references to "a/an/the element, apparatus, component, means, etc. are to be interpreted openly as referring to at least one instance of the element, apparatus, component, means, etc.", unless explicitly stated otherwise.

### BRIEF DESCRIPTION OF THE DRAWINGS

The specific embodiments of the inventive concept will now be described, by way of example, with reference to the accompanying drawings, in which:
Fig. 1 depicts a perspective view of an example of a medicament delivery training device;
Fig. 2 is an exploded view of the medicament delivery training device in Fig. 1;
Fig. 3 is a perspective view of a housing of the medicament delivery training device;
Fig. 4 is a perspective view of a housing of the medicament delivery training device when the container is fitted in the housing;
Fig. 5 is a longitudinal sectional view of a container of a medicament delivery training device;
Fig. 6 shows a perspective view of a plunger rod, an actuator sleeve and a holding structure;
Fig. 7 shows a perspective view of a cap with a resetting structure;
Fig. 8 is a longitudinal section of the medicament delivery training device in Fig. 1 in an initial state;
Fig. 9 is a longitudinal section of the medicament delivery training device with the cap removed;
Fig. 10 is a longitudinal section of the medicament delivery training device when the medicament delivery training device is being activated;
Fig. 11 is a longitudinal section of the medicament delivery training device when the plunger rod has been released;
Fig. 12 is a longitudinal section of the medicament delivery training device just before the plunger rod has moved maximally in the proximal direction;
Fig. 13 is a longitudinal section of the medicament delivery training device when the plunger rod has reached its most proximal position;
Fig. 14 is a longitudinal section of the medicament delivery training device when the delivery member cover has been released;
Fig. 15 is a longitudinal section of the medicament delivery training device when it is being reset; and

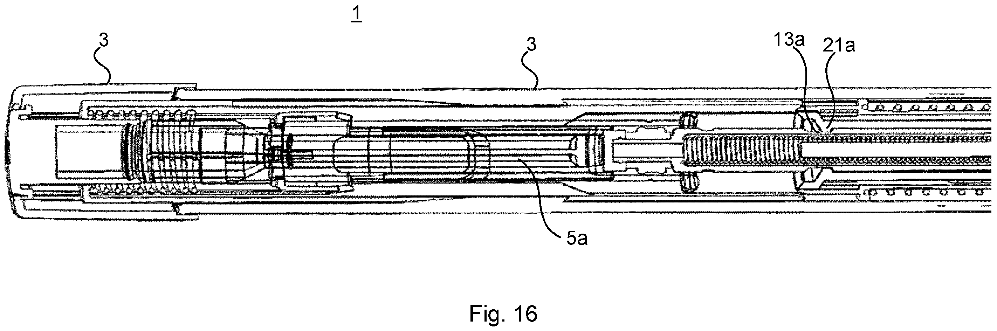

Fig. 16 is a longitudinal section of the medicament delivery training device when the medicament delivery training device has been reset.

### DETAILED DESCRIPTION

The inventive concept will now be described more fully hereinafter with reference to the accompanying drawings, in which exemplifying embodiments are shown. The inventive concept may, however, be embodied in many different forms and should not be construed as limited to the embodiments set forth herein; rather, these embodiments are provided by way of example so that this disclosure will be thorough and complete, and will fully convey the scope of the inventive concept to those skilled in the art. Like numbers refer to like elements throughout the description.

In the present disclosure, when the term "distal" is used, this refers to the direction pointing away from the dose delivery site. When the term "distal part/end" is used, this refers to the part/end of the medicament delivery training device, or the parts/ends of the members thereof, which under use of the medicament delivery training device is/are located furthest away from the dose delivery site. Correspondingly, when the term "proximal" is used, this refers to the direction pointing to the dose delivery site. When the term "proximal part/end" is used, this refers to the part/end of the medicament delivery training device, or the parts/ends of the members thereof, which under use of the medicament delivery training device is/are located closest to the dose delivery site.

Further, the term "longitudinal", with or without "axis", refers to a direction or an axis through the device or components thereof in the direction of the longest extension of the device and/or component.

Similarly, the terms "radial", "transversal" or "orthogonal" refers to a direction generally perpendicular to the longitudinal direction which is the axis direction and e.g. "radially or orthogonally outward" will refer to a direction pointing away for the longitudinal axis.

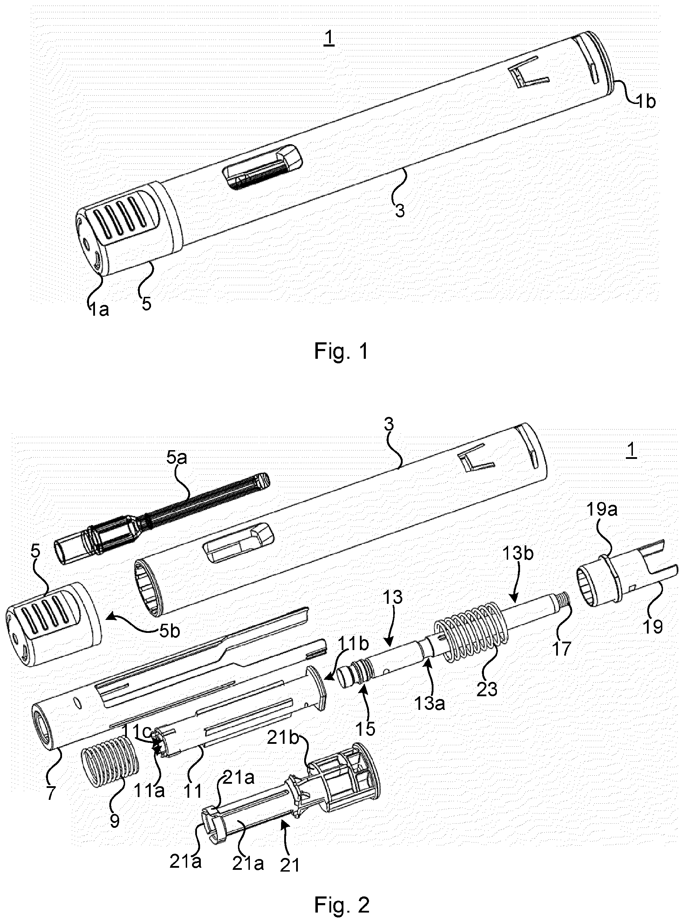

Fig. 1 depicts an example of a medicament delivery training device 1. The medicament delivery training device 1 is a dummy device to be used for learning how a corresponding real medicament delivery device is to be handled and operated.

The medicament delivery training device 1 has a proximal end 1a and a distal end 1b. The medicament delivery training device 1 comprises a housing 3 and a cap 5. The cap 5 is configured to be removably attached to the housing 3. The cap 5 forms the proximal end 1a of the medicament delivery training device 1 when attached to the housing 3.

Fig. 2 shows an exploded view of the medicament delivery training device 1. The exemplified medicament delivery training device 1 comprises a resetting structure 5a. The resetting structure 5a has an elongated shape. The resetting structure 5a may comprise a rod. The resetting structure 5a is configured to be attached to the cap 5. The cap 5 has a distal opening 5b configured to receive the resetting structure 5a. The resetting structure 5a extends in the distal direction from the cap 5 when fitted in the cap 5, along a central axis of the cap 5.

The medicament delivery training device 1 comprises a delivery member cover 7, and a first resilient member 9 configured to bias the delivery member cover 7 in the proximal direction. The exemplified first resilient member 9 may be a spring. The delivery member cover 7 is configured to be received by the housing 3. The delivery member cover 7 is configured to be moved linearly relative to the housing 3, between an initial position and an activation position. The delivery member cover 7 extends more in the proximal direction from the housing 3 in the initial position than in the activation position. Hereto, the initial position is an extended position and the activation position is a retracted position relative to the housing 3. The first resilient member 9 is configured to bias the delivery member cover 7 towards the initial position, i.e. the extended position.

The medicament delivery training device 1 comprises a container 11. The container 11 is a syringe dummy. Since it is the syringe dummy, the exemplified container 11 does not comprise any needle or canula. The container 11 has an inner chamber 11a. The inner chamber 11a extends through the container 11. The container 11 has an open distal container end 11b. The container 11 has an open proximal container end 11c. The inner chamber 11a extends between the open distal container end 11b and the open proximal container end 11c.

The medicament delivery training device 1 comprises a plunger rod 13. The plunger rod 13 is provided with a stopper 15. The stopper 15 is provided on a proximal end of the plunger rod 13. The stopper 15 may be made of an elastic, resilient or flexible material.

The plunger rod 13 may have a proximal end face or cap/cover which may form part of the stopper 15 or which may extend in the proximal direction from the stopper 15. The proximal end face of the plunger rod 13 may be made of a rigid material such as a plastic material or a metal. In examples in which the plunger rod has a proximal end face, the proximal end face and the elastic stopper body together form the stopper as defined herein.

The plunger rod 13, the stopper 15 and the container 11 forms part of a drive mechanism of the medicament delivery training device 1.

The container 11 is configured to receive the plunger rod 13. The plunger rod 13 is configured to extend in the proximal direction into the inner chamber 11a through the open distal container end 11b.

The exemplified plunger rod 13 comprises a recess 13a. The recess 13a is a radial recess. In the example shown in Fig. 2, the recess 13a extends a full turn in the circumferential direction. The plunger rod 13 could alternatively comprise a plurality of physically separate recesses distributed in the circumferential direction. The recess 13a may have an inclined distal transition surface, relative to the central axis of the plunger rod 13, where the recess 13a transitions to the outer surface of the plunger rod 13.

The medicament delivery training device 1 comprises a second resilient member 17 configured to bias the plunger rod 13 in the proximal direction. The second resilient member 17 may be a spring. The second resilient member 17 is configured to be received by the plunger rod 13. The plunger rod 13 may hence be hollow.

The medicament delivery training device 1 comprises a holding structure 21. The holding structure 21 is configured to be received by the housing 3. The holding structure 21 is configured to be fixedly arranged in the housing 3. The holding structure 21 is axially fixed relative to the housing 3. The holding structure 21 is arranged in a distal portion of the housing 3. The holding structure 21 is an elongated structure. The holding structure 21 is hollow. The holding structure 21 is configured to receive a distal end portion 13b of the plunger rod 13.

The holding structure 21 comprises a plurality of radially flexible arms 21a. The radially flexible arms 21a extend in the proximal direction. The radially flexible arms 21a form a proximal end of the holding structure 21. Each radially flexible arm 21a is configured to engage with the recess 13a of the plunger rod 13. Each radially flexible arm 21a comprises a radially inwards extending protrusion configured to engage with the recess 13a.

The medicament delivery training device 1 comprises an actuator sleeve 19. The actuator sleeve 19 is configured to be arranged around a proximal portion of the holding structure 21. The actuator sleeve 19 is configured to be moved linearly relative to the holding structure 21 between a proximal position and a distal position. The delivery member cover 7 is configured to move the actuator sleeve 19 from the proximal position to the distal position. When the delivery member cover 7 is in the initial position, the actuator sleeve 19 is arranged in the proximal position. When the delivery member cover 7 is moved in the distal direction towards the activation position, the actuation sleeve 19 is moved towards the distal position. The actuator sleeve 19 has a proximal flange 19a. The delivery member cover 7 has distally extending legs configured to bear against/engage with the proximal flange 19a when the delivery member cover 7 is moved towards the activation position. The actuation sleeve 19 is thereby moved towards the distal position as the delivery member cover 7 is moved in the distal direction.

The actuator sleeve 19 is configured to be arranged around the radially flexible arms 21a and urge the radially flexible arms 21a to engage with the recess 13a of the plunger rod 13 when the actuator sleeve 19 is in the proximal position.

The medicament delivery training device 1 comprises a third resilient member 23. The third resilient member 23 may be a spring. The third resilient member 23 is configured to bias the actuator sleeve 19 in the proximal direction towards the proximal position. The holding member 21 has a distal flange surface 21b. The third resilient member 23 is configured to be arranged between the distal end of the proximal flange 19a of the actuator sleeve 19 and the distal flange surface 21b. The third resilient member 23 thereby biases the actuator sleeve 19 in the proximal direction towards the proximal position.

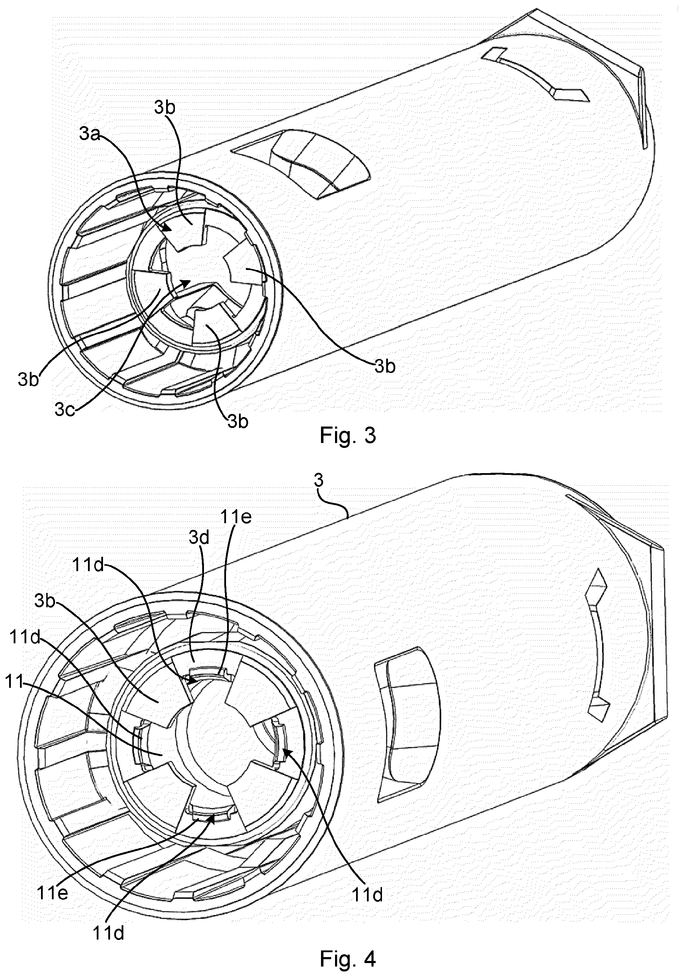

Fig. 3 shows a perspective view of the housing 3. The housing 3 has an inner stop structure 3a. The stop structure 3a comprises a plurality of radially inwards extending protrusions 3b. The radially inwards extending protrusions 3b form a central proximal opening 3c between them. The radially inwards extending protrusions 3b do hence not meet as they extend towards the central axis of the housing 3. The central proximal opening 3c is dimensioned such that the resetting structure 5a may be received through the central proximal opening 3c when the cap 5 with the resetting structure 5a is attached to the housing 3 and hence the resetting structure 5a is inserted into the housing 3. The central proximal opening 3c is dimensioned such that the stopper 15 is not able to move through the central proximal opening 3c when the plunger rod 13 is moved in the proximal direction, as will be explained in what follows. Hereto, the stopper 15 has an outer stopper diameter which is larger than the central proximal opening 3c.

Fig. 4 shows a perspective view of the housing 3 when the container 11 is arranged in the housing 3. The container 11 is provided with a proximal engagement structure 11d configured to engage with the housing 3. The proximal engagement structure 11d comprises a plurality of radially outwards extending flexible snap structures 11e. According to the present example, the housing 3 has a radially inwards extending structure 3d between each adjacent pair of radially inwards extending protrusion 3b. The radially inwards extending structures 3d have a shorter radially inwards extension than the radially inwards extending protrusions 3b. Each of the plurality of radially outwards extending flexible snap structures 11e is configured to engage with a respective radially inwards extending structure 3d. The container 11 is thereby attached to the housing 3.

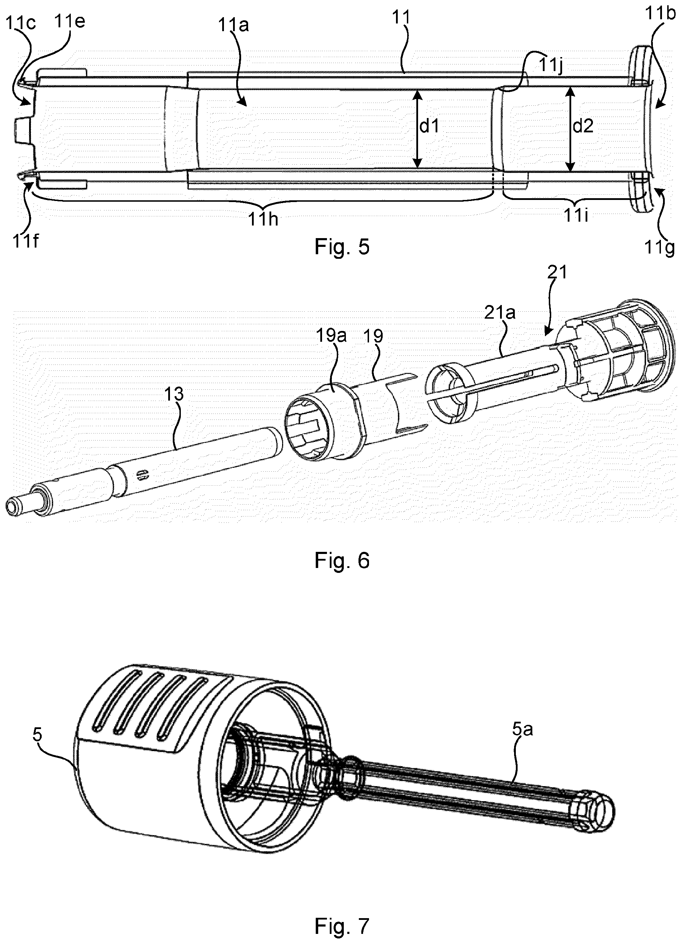

Fig. 5 shows a longitudinal sectional view of the container 11. The container 11 has a proximal container end 11f and a distal container end 11g. The inner chamber or channel 11a which extends between the proximal container end 11f and the distal container end 11g has a proximal portion 11h and a distal portion 11i. The proximal portion has a proximal portion inner diameter d1 and the distal portion 11i has a distal portion inner diameter d2. The proximal portion inner diameter d1 is smaller than the distal portion inner diameter d2.

The open distal container end 11b opens into the distal portion 11i. The distal portion 11i transitions to the proximal portion 11h via a stopper contact surface 11j. The stopper contact surface 11j may for example be inclined relative to the central longitudinal axis of the container 11, as shown in Fig. 5, or it may be orthogonal to the central longitudinal axis. In the former case, the stopper contact surface 11j provides a gradual decrease of the inner diameter of the container 11 in the proximal direction. In the latter case, the stopper contact surface forms a step between the distal portion 11i and the proximal portion 11h.

The outer stopper diameter may for example be defined by the largest outer dimension or diameter of the stopper 15. The proximal portion inner diameter d1 is smaller than the outer stopper diameter. The proximal portion inner diameter d1 is dimensioned to provide friction onto the stopper 15 to control the driving speed of the plunger rod 13. The driving speed should preferably simulate the driving speed of the plunger rod of the corresponding real medicament delivery device which the medicament delivery training device 1 is designed to simulate.

Fig. 6 shows a perspective view of the plunger rod 13, the actuator sleeve 19 and the holding structure 21.

Fig. 7 is a perspective view of the cap 5 with the resetting structure 5a attached to the cap 5. In a typical example, the resetting structure 5a is fixedly attached to the cap 5 such that when the cap 5 is removed from the housing 3, the resetting structure 5a is also removed from the housing 3 together with the cap 5.

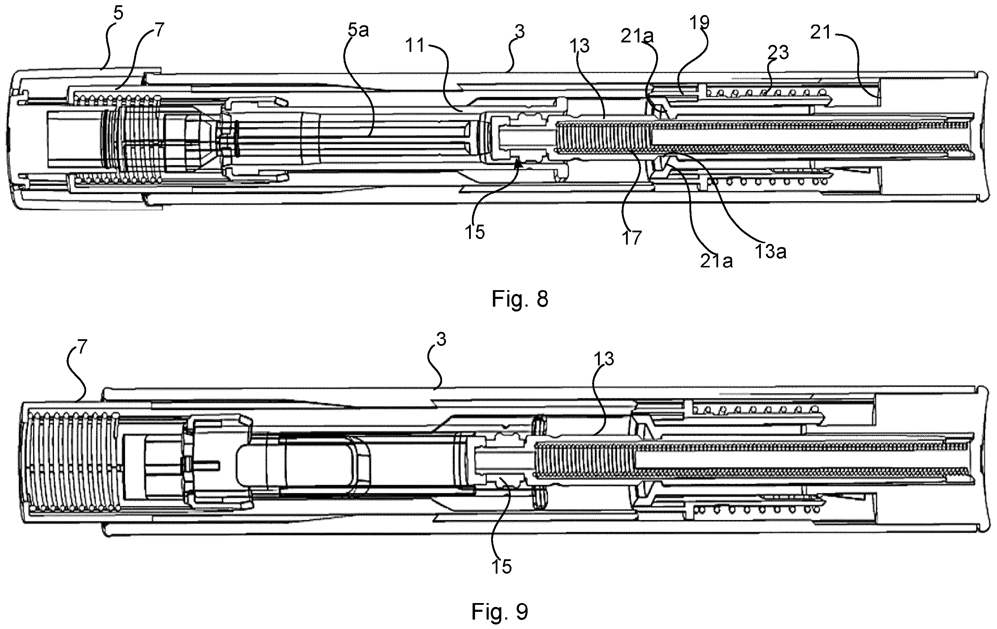

Fig. 8 shows a longitudinal section of the medicament delivery training device 1 in an initial state thereof. The initial state is the state of the medicament delivery training device 1 before it has been activated. In the initial state, the cap 5 is attached to the housing 3. The resetting structure 5a extends into the container 11 through the central proximal opening 3c of the housing 3. The drive mechanism is also in an initial state when the medicament delivery training device 1 is in the initial state.

The actuator sleeve 19 is arranged in the proximal position as it is biased by the third resilient member 23 in the proximal direction and the delivery member cover 7 is in the initial position in which it does not apply any, or at least negligible force in the distal direction onto the actuator sleeve 19. The radially flexible arms 21a are hence urged radially inwards by the actuator sleeve 19 which is arranged around the radially flexible arms 21a. The radially flexible arms 21a thereby engage with the recess 13a. The plunger rod 13 is therefore maintained in a first position in which it extends through the open distal container end 11b and the stopper 15 is arranged fixed in the distal portion 11i of the container 11. The proximal end of the stopper 15 is typically arranged at a distance from the stopper contact surface 11j.

When a user is to perform training with the medicament delivery training device 1, the cap 5 is first to be removed from the housing 3. This is shown in Fig. 9. As the cap 5 is removed, so is the resetting structure 5a. The delivery member cover 7 is in the initial position, i.e. in the extended position relative to the housing 3. The plunger rod 13 is still fixed in the first position as in the initial state depicted in Fig. 8.

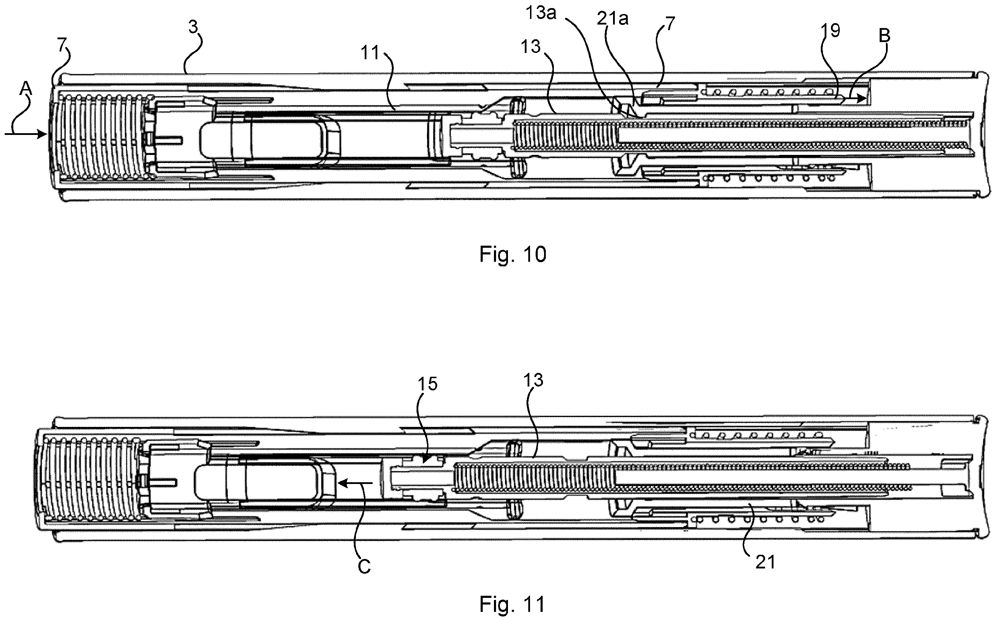

In Fig. 10, the delivery member cover 7 has been moved into the housing 3 to the activation position as shown by arrow A. This may for example be achieved by a user pressing the medicament delivery training device 1 towards an intended site of injection. The delivery member cover 7 has pushed the actuation sleeve 19 in the distal direction towards the distal position, as shown by arrow B. The actuation sleeve 19 is hence moved distally from the radially flexible arms 21a. Since the plunger rod 13 is biased in the proximal direction and urged towards the proximal container end 11c, the radially flexible arms 21a are now moved out from the recess 13a. The plunger rod 13 is hence released and moved in the proximal direction further into the container 11.

Fig. 11 shows the medicament delivery training device 1 when the plunger rod 13 has been released from its engagement with the holding structure 21. The stopper 15 has moved from the distal portion 11i into the proximal portion 11h of the inner chamber 11a via the stopper contact surface 11j, as shown by arrow C. As the stopper 15 is moved from the distal portion 11i into the proximal portion 11h, the stopper impacts with the stopper contact surface 11j, since the outer stopper diameter is larger than the proximal portion inner diameter d1. This impact results in an initial signal. The initial signal may be an audible sound such as a click. A user will thereby become aware that simulated medicament administration has commenced.

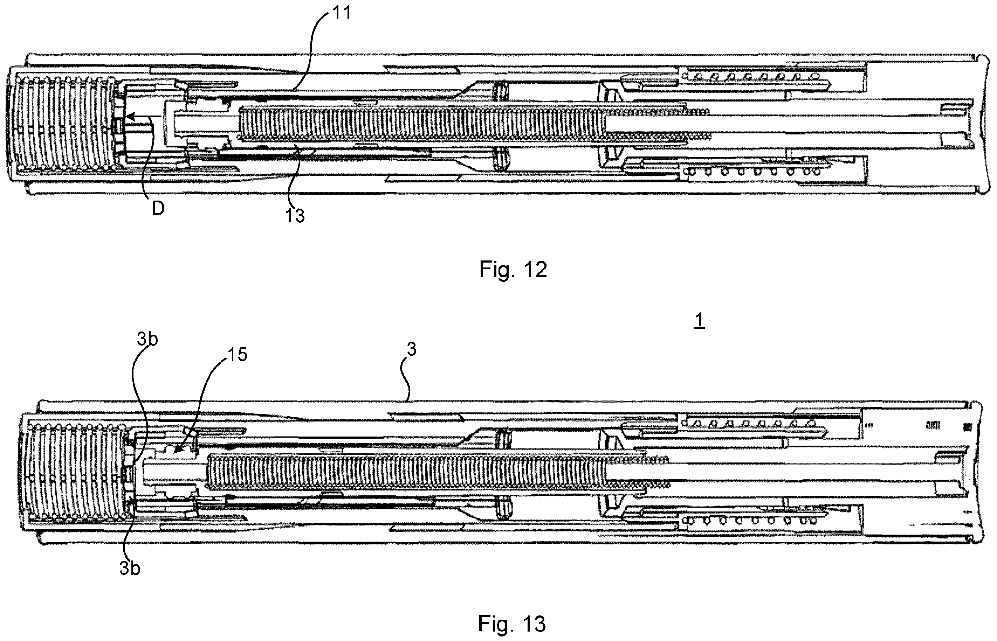

In Fig. 12 the plunger rod 13 has been further moved in the proximal direction inside the container 11 as shown by arrow D.

In Fig. 13, the stopper 15 has impacted with the housing 3, in particular with the inner stop structure 3a with the plurality of radially inwards extending protrusions 3b. A subsequent signal is generated by this impact. The subsequent signal may for example be an audible sound such as a click. A user of the medicament delivery training device 1 will thereby understand that the simulated medicament injection has been finalised.

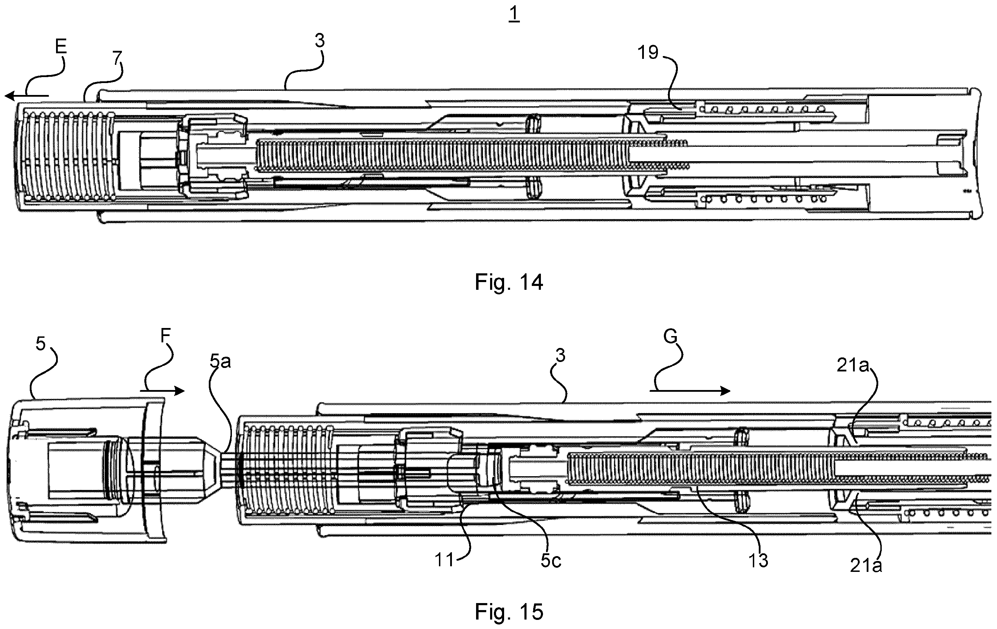

In Fig. 14 the medicament delivery training device 1 has been removed from the injection site by the user. The delivery member cover 7 is hence moved back to the initial position due to its proximal biasing, as shown by arrow E. As a result, the actuation sleeve 19 is moved back to the proximal position from the distal position.

In this used state of the medicament delivery training device 1, the medicament delivery training device 1 may be reset manually for further training. The plunger rod 13 may in particular be reset to the first position.

This resetting may be performed by manually inserting the resetting structure 5a. In particular, the resetting may be performed manually by a user placing the cap 5 back onto the housing 3.

Fig. 15 shows a resetting operation of the medicament delivery training device 1. The cap 5 is being placed back onto the housing 3 as shown by arrow F. The resetting structure 5a thereby first penetrates the central proximal opening 3c formed between the radially inwards extending protrusions 3b and subsequently the open proximal container end 11c of the container 11. The resetting structure 5a has a distal end tip 5c which pushes the plunger rod 13 in the distal direction towards the first position, as shown by arrow G. The resetting structure 5a has a length which enables it to reset the plunger rod 13 in the first position when the cap 5 is attached to the housing 3. Hence, as the resetting structure 5a is moved further into the container 11 the recess 13a of the plunger rod 13 will become aligned with the radially flexible arms 21a, which at this time are urged radially inwards by the actuator sleeve 19 arranged in the proximal position and hence around the radially flexible arms 21a. The radially flexible arms 21a will therefore engage with the recess 13a and the plunger rod 13 once again becomes fixedly arranged in the first position with the stopper 15 arranged in the distal portion 11i of the container 11. This is shown in Fig. 15, where the medicament delivery training device 1 has once again obtained its initial state in which it is ready to be used for further training.

The inventive concept has mainly been described above with reference to a few examples. However, as is readily appreciated by a person skilled in the art, other embodiments than the ones disclosed above are equally possible within the scope of the inventive concept, as defined by the appended claims.

## Claims

1. A drive mechanism for a medicament delivery training device (1), the delivery mechanism comprising:
a plunger rod (13) having a proximal end and a distal end, wherein the proximal end is pointing towards a delivery site,
a stopper (15) provided on the proximal end of the plunger rod (13), and
a container (11) having an inner chamber (11a), a proximal container end (11c), and an open distal container end (11b) leading into the inner chamber (11a), wherein the inner chamber (11a) has a distal portion (11i) and a proximal portion (11h),
wherein the stopper (15) has an outer stopper diameter, the distal portion (11i) has a distal portion inner diameter (d2) and the proximal portion (11h) has a proximal portion inner diameter (d1) which is smaller than the distal portion inner diameter (d2) and the outer stopper diameter, the distal portion (11i) transitioning to the proximal portion (11h) via a stopper contact surface (11j), wherein the drive mechanism is configured such that
in an initial state of the delivery mechanism the plunger rod (13) is axially fixedly arranged in a first position in which the stopper (15) is arranged distally relative to the proximal portion (11h), and the plunger rod (13) is biased towards the proximal container end (11c), and wherein the plunger rod (13) is configured to be released from the first position, causing the plunger rod (13) to move towards the proximal container end (11c) and the stopper (15) to impact with the stopper contact surface (11j), and to subsequently move into the proximal portion (11h), **characterised in that** the stopper (15) and the stopper contact surface (11j) are configured such that said impact results in an initial signal.

2. The drive mechanism as claimed in claim 1, comprising a holding structure (21) provided with a radially flexible arm (21a), wherein the plunger rod (13) is provided with a radial recess (13a) configured to engage with the radially flexible arm (21a) to maintain the plunger rod (13) axially fixed in the distal portion (11i).

3. The drive mechanism as claimed in claim 2, comprising an actuator sleeve (19) configured to receive the holding structure (21), the actuator sleeve (19) being configured to move between a proximal position and a distal position, wherein in the proximal position the actuator sleeve (19) is configured to urge the radially flexible arm (21a) radially inwards to engage with the radial recess (13a).

4. The drive mechanism as claimed in claim 3, wherein when the actuator sleeve (19) is in the distal position, the actuator sleeve (19) is configured to enable the radially flexible arm (21a) to move radially outwards to disengage from the radial recess (13a), thereby releasing the plunger rod (13) from the first position.

5. The drive mechanism as claimed in claim 3 or 4, wherein the actuator sleeve (19) is biased towards the proximal position.

6. The drive mechanism as claimed in any of claims 3 to 5, comprising a delivery member cover (7) configured to move linearly relative to the container (11), between an initial position and an activating position, wherein the delivery member cover (7) is configured to move the actuator sleeve (19) from the proximal position to the distal position when moved from the initial position to the activating position.

7. The drive mechanism as claimed in any of the preceding claims, comprising a housing (3) configured to receive the container (11), wherein the housing (3) has an inner stop structure (3a) configured to stop proximal movement of the plunger rod (13) in the inner chamber (11a), causing the stopper (15) to impact with the stop structure (3a), resulting in a subsequent signal.

8. The drive mechanism as claimed in claim 7, wherein the stop structure (3a) comprises a plurality of radially inwards extending protrusions (3b).

9. The drive mechanism as claimed in claim 8, wherein the container (11) is provided with a proximal engagement structure (11d) configured to engage with the housing (3).

10. The drive mechanism as claimed in claim 8 or 9, wherein the radially inwards extending protrusions (3b) are arranged to form a central proximal opening (3c) into the container (11).

11. The drive mechanism as claimed in any of the preceding claims, wherein the stopper (15) is made of a resilient material.

12. The drive mechanism as claimed in any of the preceding claims, wherein the proximal portion inner diameter (d1) is dimensioned to cause friction between the stopper (15) and the inner surface of the proximal portion to control a driving speed of the plunger rod (13).

13. A medicament delivery training device (1) comprising a delivery mechanism as claimed in any of claims 1-12.

14. The medicament delivery training device (1) as claimed in claim 13, wherein the drive mechanism is configured to be actuated mechanically or electrically.

## Patentansprüche

1. Antriebsmechanismus für eine Medikamentenabgabetrainingsvorrichtung (1), der Abgabemechanismus umfassend:
eine Kolbenstange (13), die ein proximales und ein distales Ende aufweist, wobei das proximale Ende zu einer Abgabestelle weist,
einen Stopper (15), der an dem proximalen Ende der Kolbenstange (13) bereitgestellt ist, und
einen Behälter (11), der eine Innenkammer (11a), ein proximales Behälterende (11c) und ein offenes distales Behälterende (11b) aufweist, das in die Innenkammer (11a) führt, wobei die Innenkammer (11a) einen distalen Abschnitt (11i) und einen proximalen Abschnitt (11h) aufweist,
wobei der Stopper (15) einen Stopperaußendurchmesser aufweist, der distale Abschnitt (11i) einen Innendurchmesser (d2) des distalen Abschnitts aufweist und der proximale Abschnitt (11h) einen Innendurchmesser (d1) des proximalen Abschnitts aufweist, der kleiner als der Innendurchmesser (d2) des distalen Abschnitts und der Stopperaußendurchmesser ist, wobei der distale Abschnitt (11i) über eine Stopperkontaktoberfläche (11j) in den proximalen Abschnitt (11h) übergeht, wobei der Antriebsmechanismus derart konfiguriert ist, dass in einem anfänglichen Zustand des Abgabemechanismus die Kolbenstange (13) axial fest in einer ersten Position angeordnet ist, in der der Stopper (15) distal relativ zu dem proximalen Abschnitt (11h) angeordnet ist, und die Kolbenstange (13) zu dem proximalen Behälterende (11c) vorgespannt ist, und wobei die Kolbenstange (13) konfiguriert ist, um aus der ersten Position freigegeben zu werden, wobei bewirkt wird, dass sich die Kolbenstange (13) zu dem proximalen Behälterende (11c) bewegt und der Stopper (15) auf die Stopperkontaktoberfläche (11j) aufprallt, und um sich anschließend in den proximalen Abschnitt (11h) zu bewegen,
**dadurch gekennzeichnet, dass** der Stopper (15) und die Stopperkontaktoberfläche (11j) derart konfiguriert sind, dass der Aufprall in einem ersten Signal resultiert.

2. Antriebsmechanismus nach Anspruch 1, umfassend eine Haltestruktur (21), die mit einem radial flexiblen Arm (21a) versehen ist, wobei die Kolbenstange (13) mit einer radialen Aussparung (13a) versehen ist, die konfiguriert ist, um den radial flexiblen Arm (21a) in Eingriff zu nehmen, um die Kolbenstange (13) axial fixiert in dem distalen Abschnitt (11i) beizubehalten.

3. Antriebsmechanismus nach Anspruch 2, umfassend eine Betätigungshülse (19), die konfiguriert ist, um die Haltestruktur (21) aufzunehmen, wobei die Betätigungshülse (19) konfiguriert ist, um sich zwischen einer proximalen Position und einer distalen Position zu bewegen, wobei in der proximalen Position die Betätigungshülse (19) konfiguriert ist, um den radial flexiblen Arm (21a) radial nach innen zu drücken, um die radiale Aussparung (13a) in Eingriff zu nehmen.

4. Antriebsmechanismus nach Anspruch 3, wobei, wenn die Betätigungshülse (19) in der distalen Position ist, die Betätigungshülse (19) konfiguriert ist, um dem radial flexiblen Arm (21a) zu ermöglichen, sich radial nach außen zu bewegen, um sich aus der radialen Aussparung (13a) zu lösen, wobei dadurch die Kolbenstange (13) aus der ersten Stellung freigegeben wird.

5. Antriebsmechanismus nach Anspruch 3 oder 4, wobei die Betätigungshülse (19) zu der proximalen Position vorgespannt ist.

6. Antriebsmechanismus nach einem der Ansprüche 3 bis 5, umfassend eine Abgabeelementabdeckung (7), die konfiguriert ist, um sich relativ zu dem Behälter (11) zwischen einer anfänglichen Position und einer Aktivierungsposition linear zu bewegen, wobei die Abgabeelementabdeckung (7) konfiguriert ist, um die Betätigungshülse (19) von der proximalen Position in die distale Position zu bewegen, wenn sie von der anfänglichen Position in die Aktivierungsposition bewegt wird.

7. Antriebsmechanismus nach einem der vorstehenden Ansprüche, umfassend ein Gehäuse (3), das konfiguriert ist, um den Behälter (11) aufzunehmen, wobei das Gehäuse (3) eine innere Stoppstruktur (3a) aufweist, die konfiguriert ist, um eine proximale Bewegung der Kolbenstange (13) in der Innenkammer (11a) zu stoppen, wobei bewirkt wird, dass der Stopper (15) auf die Stoppstruktur (3a) aufprallt, was in einem anschließenden Signal resultiert.

8. Antriebsmechanismus nach Anspruch 7, wobei die Stoppstruktur (3a) eine Vielzahl von sich radial nach innen erstreckenden Vorsprüngen (3b) aufweist.

9. Antriebsmechanismus nach Anspruch 8, wobei der Behälter (11) mit einer proximalen Eingriffsstruktur (11d) versehen ist, die konfiguriert ist, um das Gehäuse (3) in Eingriff zu nehmen.

10. Antriebsmechanismus nach Anspruch 8 oder 9, wobei die sich radial nach innen erstreckenden Vorsprünge (3b) angeordnet sind, um eine zentrale proximale Öffnung (3c) in den Behälter (11) auszubilden.

11. Antriebsmechanismus nach einem der vorstehenden Ansprüche, wobei der Stopper (15) aus einem elastischen Material hergestellt ist.

12. Antriebsmechanismus nach einem der vorstehenden Ansprüche, wobei der Innendurchmesser (d1) des proximalen Abschnitts bemessen ist, um zwischen dem Stopper (15) und der Innenoberfläche des proximalen Abschnitts Reibung zu bewirken, um eine Antriebsgeschwindigkeit der Kolbenstange (13) zu steuern.

13. Medikamentenabgabetrainingsvorrichtung (1), umfassend einen Abgabemechanismus nach einem der Ansprüche 1 bis 12.

14. Medikamentenabgabetrainingsvorrichtung (1) nach Anspruch 13, wobei der Antriebsmechanismus konfiguriert ist, um mechanisch oder elektrisch betätigt zu werden.

## Revendications

1. Mécanisme d'entraînement pour un dispositif d'apprentissage d'administration de médicaments (1), le mécanisme d'administration comprenant :
une tige de piston (13) ayant une extrémité proximale et une extrémité distale, dans lequel l'extrémité proximale est orientée vers un site d'administration,
un élément de butée (15) placé sur l'extrémité proximale de la tige de plongeur (13), et
un récipient (11) ayant une chambre intérieure (11a), une extrémité de récipient proximale (11c) et une extrémité de récipient distale ouverte (11b) menant dans la chambre intérieure (11a), dans lequel la chambre intérieure (11a) a une partie distale (11i) et une partie proximale (11h),
dans lequel l'élément de butée (15) a un diamètre d'élément de butée extérieur, la partie distale (11i) a un diamètre intérieur de partie distale (d2) et la partie proximale (11h) a un diamètre intérieur de partie proximale (d1) qui est plus petit que le diamètre intérieur de partie distale (d2) et le diamètre d'élément de butée extérieur, la partie distale (11i) passant à la partie proximale (11h) par une surface de contact d'élément de butée (11j), dans lequel le mécanisme d'entraînement est conçu de telle sorte que, dans un état initial du mécanisme d'administration, la tige de piston (13) est agencée axialement de manière fixe dans une première position dans laquelle l'élément de butée (15) est agencé de manière distale par rapport à la partie proximale (11h), et la tige de piston (13) est sollicitée vers l'extrémité de récipient proximale (11c), et dans lequel la tige de piston (13) est conçue pour être libérée de la première position, amenant la tige de piston (13) à se déplacer vers l'extrémité de récipient proximale (11c) et l'élément de butée (15) à percuter la surface de contact d'élément de butée (11j), et ensuite à se déplacer dans la partie proximale (11h) ;
**caractérisé en ce que** l'élément de butée (15) et la surface de contact d'élément de butée (11j) sont conçus de telle sorte que ladite percussion donne lieu à un signal initial.

2. Mécanisme d'entraînement selon la revendication 1, comprenant une structure de support (21) pourvue d'un bras radialement flexible (21a), dans lequel la tige de piston (13) est pourvue d'un renfoncement radial (13a) conçu pour venir en prise avec le bras radialement flexible (21a) afin de maintenir la tige de piston (13) axialement fixe dans la partie distale (11i).

3. Mécanisme d'entraînement selon la revendication 2, comprenant un manchon d'actionnement (19) conçu pour recevoir la structure de support (21), le manchon d'actionnement (19) étant conçu pour se déplacer entre une position proximale et une position distale, dans lequel, dans la position proximale, le manchon d'actionnement (19) est conçu pour pousser le bras radialement flexible (21a) radialement vers l'intérieur pour venir en prise dans le renfoncement radial (13a).

4. Mécanisme d'entraînement selon la revendication 3, dans lequel, lorsque le manchon d'actionnement (19) est dans la position distale, le manchon d'actionnement (19) est conçu pour permettre au bras radialement flexible (21a) de se déplacer radialement vers l'extérieur pour se désaccoupler du renfoncement radial (13a), libérant ainsi la tige de plongeur (13) de la première position.

5. Mécanisme d'entraînement selon la revendication 3 ou 4, dans lequel le manchon d'actionnement (19) est sollicité vers la position proximale.

6. Mécanisme d'entraînement selon l'une quelconque des revendications 3 à 5, comprenant un couvercle d'élément d'administration (7) conçu pour se déplacer linéairement par rapport au récipient (11), entre une position initiale et une position d'activation, dans lequel le couvercle d'élément d'administration (7) est conçu pour déplacer le manchon d'actionnement (19) de la position proximale à la position distale lorsqu'il est déplacé de la position initiale à la position d'activation.

7. Mécanisme d'entraînement selon l'une quelconque des revendications précédentes, comprenant un boîtier (3) conçu pour recevoir le récipient (11), dans lequel le boîtier (3) a une structure de butée intérieure (3a) conçue pour arrêter un mouvement proximal de la tige de plongeur (13) dans la chambre intérieure (11a), amenant l'élément de butée (15) à percuter la structure de butée (3a), ce qui entraîne un signal ultérieur.

8. Mécanisme d'entraînement selon la revendication 7, dans lequel la structure de butée (3a) comprend une pluralité de saillies (3b) s'étendant radialement vers l'intérieur.

9. Mécanisme d'entraînement selon la revendication 8, dans lequel le récipient (11) est pourvu d'une structure de mise en prise proximale (11d) conçue pour venir en prise dans le boîtier (3).

10. Mécanisme d'entraînement selon la revendication 8 ou 9, dans lequel les saillies (3b) s'étendant radialement vers l'intérieur sont agencées de manière à former une ouverture centrale proximale (3c) dans le récipient (11).

11. Mécanisme d'entraînement selon l'une quelconque des revendications précédentes, dans lequel l'élément de butée (15) est constitué d'un matériau élastique.

12. Mécanisme d'entraînement selon l'une quelconque des revendications précédentes, dans lequel le diamètre intérieur de partie proximale (d1) est dimensionné pour amener un frottement entre l'élément de butée (15) et la surface intérieure de la partie proximale afin de commander une vitesse d'entraînement de la tige de plongeur (13).

13. Dispositif d'apprentissage d'administration de médicaments (1) comprenant un mécanisme d'administration selon l'une quelconque des revendications 1 à 12.

14. Dispositif d'apprentissage d'administration de médicaments (1) selon la revendication 13, dans lequel le mécanisme d'entraînement est conçu pour être actionné mécaniquement ou électriquement.
